# EUROPEAN PATENT APPLICATION

(11) **EP 3 476 841 A1**
(43) Date of publication of application: **01.05.2019**
(21) Application number: 17814745.0
(22) Date of filing: 23.06.2017
(51) Int. Cl.: C07D 403/04, A61K 31/506, A61P 35/00, A61P 35/02

(54) **CRYSTALS OF ANILINE PYRIMIDINE COMPOUND SERVING AS EGFR INHIBITOR**

(30) Priority: 24.06.2016 CN 201610470835
(71) Applicant: Chia Tai Tianqing Pharmaceutical Group Co., Ltd., Lianyungang, Jiangsu 222062 (CN); Lianyungang Runzhong Pharmaceutical Co., Ltd., Lianyungang, Jiangsu 222069 (CN); Centaurus BioPharma Co., Ltd., Beijing 100195 (CN)
(72) Inventor: ZHU, Yizhong, Lianyungang Jiangsu 222062 (CN); TANG, Jianqiu, Lianyungang Jiangsu 222062 (CN); LIU, Fei, Lianyungang Jiangsu 222062 (CN); ZHANG, Xiquan, Lianyungang Jiangsu 222062 (CN); GU, Hongmei, Lianyungang Jiangsu 222062 (CN); ZHU, Bo, Lianyungang Jiangsu 222062 (CN); WANG, Lulu, Lianyungang Jiangsu 222062 (CN); TANG, Song, Lianyungang Jiangsu 222062 (CN); ZHANG, Yanyang, Lianyungang Jiangsu 222062 (CN)
(74) Representative: Gill Jennings & Every LLP
(86) International application number: PCT/CN2017/089693
(87) International publication number: WO 2017/220007

(57) **Abstract**

The present application belongs to the field of medicinal chemistry, and relates to crystals of an aniline pyrimidine compound serving as an EGFR inhibitor. Specifically, the present application relates to crystal A, crystal B and crystal C of N-(2-((2-(dimethyl amino)ethyl)(methyl)amino)-4-methoxy-5-(4-(3-methyl-2-oxo-2,3-dihydro-1H-benzo[d]imidazole-1-base)pyrimidine-2-base amino)phenyl)acrylamide (formula I) hydrochloride, and also relates to the method for preparing the crystal A, the crystal B and the crystal C, a crystal composition comprising the crystal A, the crystal B, and the crystal C, a pharmaceutical composition comprising the crystal A, the crystal B and the crystal C or the crystal composition thereof, and medical uses thereof. The crystal A, the crystal B and the crystal C in the present application has the advantages of high purity, high crystallization degree, good stability and the like.

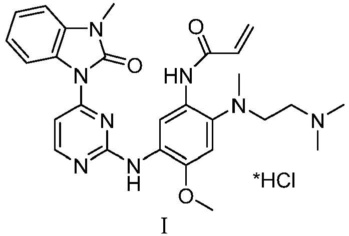

## Description

### CROSS-REFERENCE TO RELATED APPLICATION

The present application claims the priority and benefit of the Chinese Patent Application No. 201610470835.2 filed at the China National Intellectual Property Administration on June 24, 2016, the disclosure of which is incorporated herein by reference in its entirety.

### TECHNICAL FIELD

The present application belongs to the field of medicinal chemistry. In particular, the present application relates to crystals of an aniline pyrimidine compound N-(2-((2-(dimethylamino)ethyl)(methyl)amino)-4-methoxy-5-(4-(3-methyl-2-oxo-2,3-dihydro-1*H*-benzo[d]imidazol-1-yl)pyrimidin-2-ylamino)phenyl)acrylamide hydrochloride as an EGFR inhibitor, crystalline compositions, pharmaceutical compositions, preparation methods and uses thereof.

### BACKGROUND ART

EGFR (Epidermal Growth Factor Receptor), also known as HER1 or ErbB1, is a receptor for cell proliferation and signal transduction of the epithelial growth factor (EGF). EGFR belongs to a member of the ErbB receptor family which includes EGFR (ErbB-1), HER2/c-neu (ErbB-2), HER3 (ErbB-3) and HER4 (ErbB-4). EGFR is a transmembrane glycoprotein with a molecular weight of 170KDa, which belongs to a tyrosine kinase receptor.

EGFR is located on the surface of cell membranes and is activated by binding to ligands including EGF and TGFα. Upon being activated, EGFR undergoes a transition from a monomer to a dimer. The dimer includes not only the binding of two identical receptor molecules (homodimerization) but also the binding of different members of the human EGF-associated receptor (HER) tyrosine kinase family (heterodimerization). EGFR can activate its intracellular kinase pathways after dimerization, resulting in the phosphorylation of key tyrosine residues in the intracellular domain and the stimulation to many intracellular signaling pathways involved in cell proliferation and survival.

There exist high or abnormal expressions of EGFR in many solid tumors. EGFR is associated with tumor cell proliferation, angiogenesis, tumor invasion, metastasis and the inhibition of apoptosis. Possible mechanisms include the followings: enhanced downstream signal transduction caused by the high expressions of EGFR; the sustained activation of EGFR caused by the increased expressions of mutant EGFR receptors or ligands; the enhanced effect of autocrine loops; the destruction of receptor downregulation mechanisms; and the activation of aberrant signaling pathways, etc. Overexpressions of EGFR play an important role in the progression of malignant tumors. Overexpressions of EGFR have been found in gliocyte, kidney cancer, lung cancer, prostate cancer, pancreatic cancer, breast cancer and other tissues.

Aberrant expressions of EGFR and Erb-B2 play a crucial role in tumor transformation and growth. In the case of lung cancer, EGFR is expressed in 50% of non-small cell lung cancer (NSCLC) cases and its expression is associated with poor prognosis. The two factors allow EGFR and its family members to be major candidates of targeted therapy. Two types of small molecule inhibitors targeted to EGFR, gefitinib and erlotinib, were rapidly approved by the FDA of USA for the treatment of advanced NSCLC patients who have no response to traditional chemotherapy.

Early clinical data indicated that 10% of NSCLC patients have response to getifinib and erlotinib. Molecular biological analysis shows that in most cases, drug-responsive patients carry specific mutations in the EGFR-encoding genes: the deletion of amino acids at positions 747-750 in exon 19 accounts for 45% of mutations, and 10% of mutations occur in exons 18 and 20. The most common EGFR-activating mutations (L858R and delE746_A750) result in an increase in affinity for small molecule tyrosine kinase inhibitors (TKI) and a decrease in affinity for adenosine triphosphate (ATP) relative to wild type (WT) EGFR. T790M mutation is a point mutation in exon 20 of EGFR, which leads to acquired resistance to the treatment with gefitinib or erlotinib. A recent study shows that the combination of L858R and T790M mutations has a stronger affinity for ATP than L858R alone, and TKIs are ATP-competitive kinase inhibitors, and thereby resulting in a decreased binding rate between TKIs and kinase domains.

Because these mutations play an important role in the drug resistance mechanism of targeting-EGFR therapy, it is necessary to provide EGFR-L858R/T790M double mutation inhibitors for use in clinical treatment. At the same time, because the inhibition of EGFR-WT will lead to a variety of clinical toxic and side effects, it is also necessary to provide inhibitors having selectivity for EGFR in the form of active mutants (such as EGFR-L858R mutant, delE746_A750 mutant, or Exon 19-deletion EGFR mutant) and/or EGFR in the form of resistant mutants (e.g., EGFR-T790M mutant), relative to EGFR-WT, for use in clinical treatment.

At present, various EGFR selective inhibitors have been reported. The Chinese patent application No. 201510419018.X with the filing date of July 16, 2015 discloses several EGFR inhibitors (the contents of which are incorporated herein by reference in their entirety), including N-(2-((2-(dimethylamino)ethyl)(methyl)amino)-4-methoxy-5-(4-(3-methyl-2-oxo-2,3-dihydro-1H-benzo[d]imidazol-1-yl)pyrimidin-2-ylamino)phenyl)acrylamide hydrochloride represented by formula I:

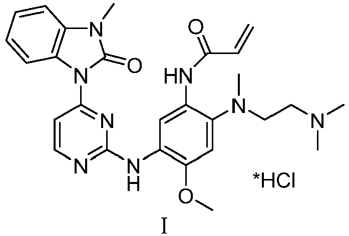

In addition to therapeutic efficacy, drug developers attempt to provide a suitable form of an active molecule having properties as a drug. From the viewpoint of obtaining a commercially viable production method or from the viewpoint of producing a pharmaceutical composition comprising an active compound, the chemical stability, solid-state stability and shelf life of an active ingredient are very important factors. Therefore, it is very important for the development of a drug to provide a suitable form of the drug having desired properties.

### SUMMARY OF THE INVENTION

In one aspect, the present application provides a crystal A of the hydrochloride of a compound represented by formula I: 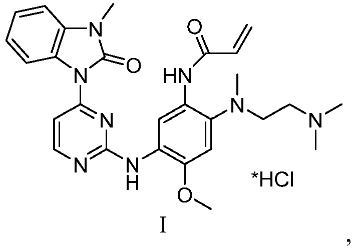 wherein an X-ray diffraction (XRD) pattern of the crystal A of the hydrochloride of the compound represented by formula I has diffraction peaks at 2θ of 8.96°±0.2°, 14.11°±0.2°, 14.87°±0.2°, 16.52°±0.2°, 18.67°±0.2°, 21.93°±0.2° and 27.09°±0.2°.

In another aspect, the present application provides a method for preparing the crystal A of the hydrochloride of the compound represented by formula I, comprising the following steps:
1) contacting the compound represented by formula I with hydrochloric acid; and
2) crystallizing the hydrochloride of the compound represented by formula I from a crystallization solvent, wherein the crystallization solvent is selected from acetonitrile, methanol, isopropanol or a mixture of ethanol and water.

In another aspect, the present application provides a crystalline composition, wherein the crystal A of the hydrochloride of the compound represented by formula I accounts for 50% or more, preferably 80% or more, more preferably 90% or more, and most preferably 95% or more, by weight of the crystalline composition.

In another aspect, the present application provides a pharmaceutical composition, wherein the pharmaceutical composition comprises a therapeutically effective amount of the crystal A of the hydrochloride of the compound represented by formula I, or the crystalline composition as described above.

In another aspect, the present application provides use of the crystal A of the hydrochloride of the compound represented by formula I or the crystalline composition or the pharmaceutical composition as described above in the preparation of a medicament for treating an EGFR-mediated disease.

In another aspect, the present application provides a crystal B of the hydrochloride of a compound represented by formula I: 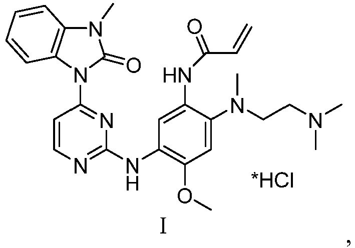 wherein an X-ray diffraction (XRD) pattern of the crystal B of the hydrochloride of the compound represented by formula I has diffraction peaks at 2θ of 9.17°±0.2°, 9.93°±0.2°, 14.07°±0.2°, 20.31°±0.2°, 21.44°±0.2° and 26.10°±0.2°.

In another aspect, the present application provides a method for preparing the crystal B of the hydrochloride of the compound represented by formula I, comprising the following steps:
1) contacting the compound represented by formula I with hydrochloric acid; and
2) crystallizing the hydrochloride of the compound represented by formula I from a crystallization solvent, wherein the crystallization solvent is ethanol.

In another aspect, the present application provides a crystalline composition, wherein the crystal B of the hydrochloride of the compound represented by formula I accounts for 50% or more, preferably 80% or more, more preferably 90% or more, and most preferably 95% or more, by weight of the crystalline composition.

In another aspect, the present application provides a pharmaceutical composition, wherein the pharmaceutical composition comprises a therapeutically effective amount of the crystal B of the hydrochloride of the compound represented by formula I, or the crystalline composition as described above.

In another aspect, the present application provides use of the crystal B of the hydrochloride of the compound represented by formula I or the crystalline composition or the pharmaceutical composition as described above in the preparation of a medicament for treating an EGFR-mediated disease.

In another aspect, the present application provides a crystal C of the hydrochloride of a compound represented by formula I: 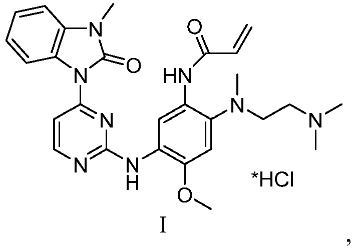 wherein an X-ray diffraction (XRD) pattern of the crystal C of the hydrochloride of the compound represented by formula I has diffraction peaks at 2θ of 7.68°±0.2°, 8.21°±0.2°, 10.89°±0.2°, 15.95°±0.2°, 19.10°±0.2°, 20.52°±0.2° and 21.54°±0.2°.

In another aspect, the present application provides a method for preparing the crystal C, comprising the following steps:
1) contacting the compound represented by formula I with hydrochloric acid; and
2) crystallizing the hydrochloride of the compound represented by formula I from a crystallization solvent, wherein the crystallization solvent is selected from tetrahydrofuran, acetone, or dioxane.

In another aspect, the present application provides a crystalline composition, wherein the crystal C of the hydrochloride of the compound represented by formula I accounts for 50% or more, preferably 80% or more, more preferably 90% or more, and most preferably 95% or more, by weight of the crystalline composition.

In another aspect, the present application provides a pharmaceutical composition, wherein the pharmaceutical composition comprises a therapeutically effective amount of the crystal C of the hydrochloride of the compound represented by formula I, or the crystalline composition as described above.

In another aspect, the present application provides use of the crystal C of the hydrochloride of the compound represented by formula I or the crystalline composition or the pharmaceutical composition as described above in the preparation of a medicament for treating an EGFR-mediated disease.

### BRIEF DESCRIPTION OF THE DRAWINGS

Fig. 1: an XRD pattern of a crystal A of the hydrochloride of a compound represented by formula I (Method 1 in Example 3).
Fig. 2: a DSC spectrum of a crystal A of the hydrochloride of a compound represented by formula I (Method 1 in Example 3).
Fig. 3: an XRD pattern of a crystal B of the hydrochloride of a compound represented by formula I (Method 5 in Example 4).
Fig. 4: a DSC spectrum of a crystal B of the hydrochloride of a compound represented by formula I (Method 5 in Example 4).
Fig. 5: an XRD pattern of a crystal C of the hydrochloride of a compound represented by formula I (Method 6 in Example 5).
Fig. 6: a DSC spectrum of a crystal C of the hydrochloride of a compound represented by formula I (Method 6 in Example 5).

### DETAILED DESCRIPTION OF THE INVENTION

In one aspect, the present application provides a crystal A of the hydrochloride of a compound represented by formula I: 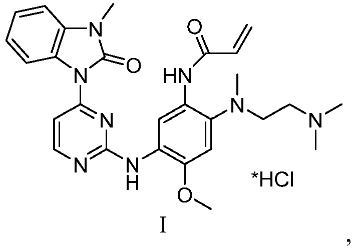 wherein an X-ray diffraction (XRD) pattern of the crystal A of the hydrochloride of the compound represented by formula I has diffraction peaks at 2θ of 8.96°, 14.11°, 14.87°, 16.52°, 18.67°, 21.93° and 27.09°±0.2°; typically has diffraction peaks at 2θ of 8.33°, 8.96°, 12.16°, 14.11°, 14.87°, 16.52°, 17.66°, 18.67°, 21.93° and 27.09°±0.2°; more typically has diffraction peaks at 2θ of 8.33°, 8.96°, 11.74°, 12.16°, 14.11°, 14.87°, 16.52°, 17.66°, 18.23°, 18.67°, 21.93°, 22.65° and 27.09°±0.2°; and further typically has diffraction peaks at 2θ of 8.33°, 8.96°, 11.74°, 12.16°, 14.11°, 14.87°, 16.52°, 17.66°, 18.23°, 18.67°, 19.48°, 19.92°, 21.93°, 22.65°, 24.95°, 27.09° and 27.55°±0.2°.

In some embodiments of the present application, X-ray diffraction peaks of the crystal A of the hydrochloride of the compound represented by formula I according to the present application have the following characteristics:

| Serial No. | 2θ±0.2 (°) | Relative Intensity (%) | Serial No. | 2θ±0.2 (°) | Relative Intensity (%) |
|---|---|---|---|---|---|
| 1 | 8.33 | 14.3 | 12 | 19.92 | 23.0 |
| 2 | 8.96 | 59.7 | 13 | 21.93 | 100.0 |
| 3 | 11.74 | 18.5 | 14 | 22.65 | 31.1 |
| 4 | 12.16 | 23.1 | 15 | 23.05 | 17.2 |
| 5 | 14.11 | 70.6 | 16 | 24.04 | 17.3 |
| 6 | 14.87 | 61.7 | 17 | 24.95 | 25.4 |
| 7 | 16.52 | 90.1 | 18 | 26.18 | 17.3 |
| 8 | 17.66 | 35.8 | 19 | 27.09 | 65.3 |
| 9 | 18.23 | 31.5 | 20 | 27.55 | 24.0 |
| 10 | 18.67 | 54.6 | 21 | 28.74 | 16.4 |
| 11 | 19.48 | 27.2 | 22 | 29.11 | 13.2 |

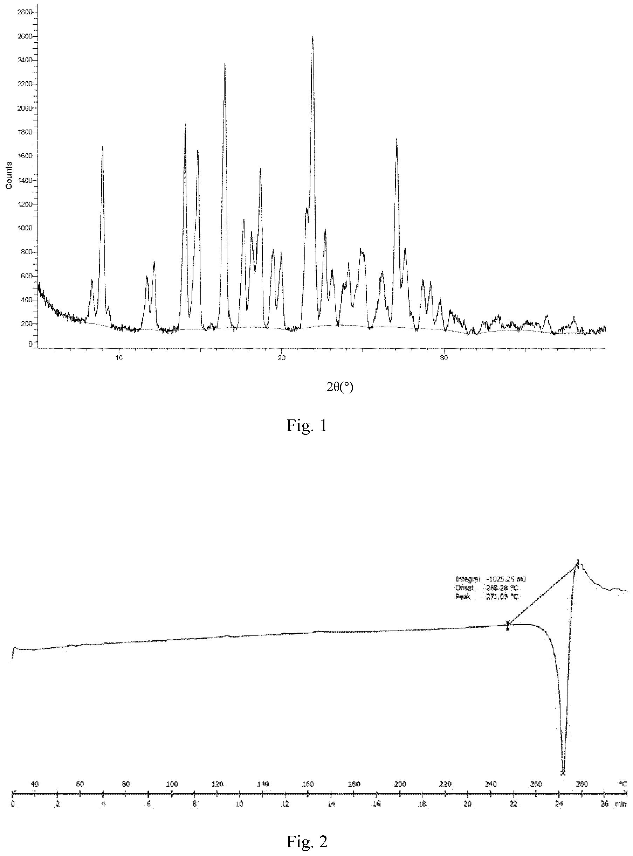

In some embodiments of the present application, an X-ray diffraction pattern of the crystal A of the hydrochloride of the compound represented by formula I according to the application is shown as Fig. 1.

In some embodiments of the present application, a DSC spectrum of the crystal A of the hydrochloride of the compound represented by formula I according to the application has a peak at about 271 °C.

In some embodiments of the present application, a DSC spectrum of the crystal A of the hydrochloride of the compound represented by formula I according to the application is shown as Fig. 2.

In another aspect, the present application provides a method for preparing the crystal A of the hydrochloride of the compound represented by formula I, comprising the following steps:
1) contacting the compound represented by formula I with hydrochloric acid; and
2) crystallizing the hydrochloride of the compound represented by formula I from a crystallization solvent, and optionally filtrating the obtained crystal;
wherein the crystallization solvent is selected from acetonitrile, methanol, isopropanol, or a mixture of ethanol and water.

In some embodiments of the present application, when the crystallization solvent for preparing the crystal A of the hydrochloride of the compound represented by formula I is a mixture of ethanol and water, the ratio of ethanol to water (by volume) is in the range of 9:1 to 1:9, preferably 9:1, 8:1, 7:1, 6:1, 5:1, 4:1, 3:1, 2:1, 1:1, 1:2, 1:3, 1:4, 1:5, 1:6, 1:7, 1:8, or 1:9, and more preferably 3:1.

In some embodiments of the present application, the molar ratio of hydrochloric acid to the compound represented by formula I in the method for preparing the crystal A of the hydrochloride of the compound represented by formula I is in the range of 1:0.5-1.5, preferably 1:0.8-1.2, and more preferably 1:1.

In some embodiments of the present application, the compound represented by formula I contacts with hydrochloric acid in the crystallization solvent.

In another aspect, the present application provides a crystalline composition of the crystal A of the hydrochloride of the compound represented by formula I. In some embodiments of the present application, the crystal A of the hydrochloride of the compound represented by formula I accounts for 50% or more, preferably 80% or more, more preferably 90% or more, and most preferably 95% or more, by weight of the crystalline composition.

In another aspect, the present application provides a pharmaceutical composition of the crystal A of the hydrochloride of the compound represented by formula I, wherein the pharmaceutical composition comprises a therapeutically effective amount of the crystal A of the hydrochloride of the compound represented by formula I, or the crystalline composition of the crystal A of the hydrochloride of the compound represented by formula I. Furthermore, the pharmaceutical composition may or may not further comprise a pharmaceutically acceptable carrier, excipient, and/or medium.

In another aspect, the present application provides use of the crystal A of the hydrochloride of the compound represented by formula I or the crystalline composition or the pharmaceutical composition as described above in the preparation of a medicament for treating an EGFR-mediated disease.

In another aspect, the present application provides a method for treating an EGFR-mediated disease, comprising administering to a mammal in need thereof a therapeutically effective amount of the crystal A of the hydrochloride of the compound represented by formula I, or the crystalline composition, or the pharmaceutical composition as described above.

In another aspect, the present application provides the crystal A of the hydrochloride of the compound represented by formula I, or the crystalline composition, or the pharmaceutical composition as described above for use in treating an EGFR-mediated disease.

In another aspect, the present application provides a crystal B of the hydrochloride of a compound represented by formula I: 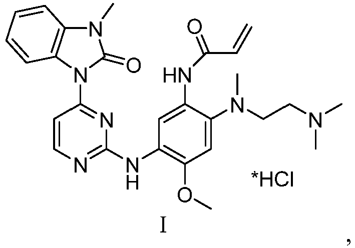 wherein an X-ray diffraction (XRD) pattern of the crystal B of the hydrochloride of the compound represented by formula I has diffraction peaks at 2θ of 9.17°, 9.93°, 14.07°, 20.31°, 21.44° and 26.10°±0.2°; typically has diffraction peaks at 2θ of 9.17°, 9.93°, 10.65°, 13.46°, 14.07°, 20.31°, 21.44°, 22.33°, 24.93° and 26.10°±0.2°; and more typically has diffraction peaks at 2θ of 6.71°, 9.17°, 9.93°, 10.65°, 11.44°, 13.46°, 14.07°, 18.94°, 20.31°, 21.44°, 21.66°, 22.33°, 24.93°, 25.73° and 26.10°±0.2°.

In some embodiments of the present application, X-ray diffraction peaks of the crystal B of the hydrochloride of the compound represented by formula I according to the present application have the following characteristics:

| Serial No. | 2θ±0.2 (°) | Relative Intensity (%) | Serial No. | 2θ±0.2 (°) | Relative Intensity (%) |
|---|---|---|---|---|---|
| 1 | 6.71 | 12.2 | 10 | 20.31 | 48.5 |
| 2 | 9.17 | 35.9 | 11 | 20.72 | 14.4 |
| 3 | 9.93 | 100.0 | 12 | 21.44 | 58.3 |
| 4 | 10.65 | 23.9 | 13 | 21.66 | 52.9 |
| 5 | 11.44 | 13.0 | 14 | 22.33 | 26.6 |
| 6 | 13.46 | 23.6 | 15 | 23.77 | 10.7 |
| 7 | 14.07 | 79.8 | 16 | 24.93 | 38.7 |
| 8 | 18.38 | 10.1 | 17 | 25.73 | 39.5 |
| 9 | 18.94 | 18.5 | 18 | 26.10 | 57.2 |

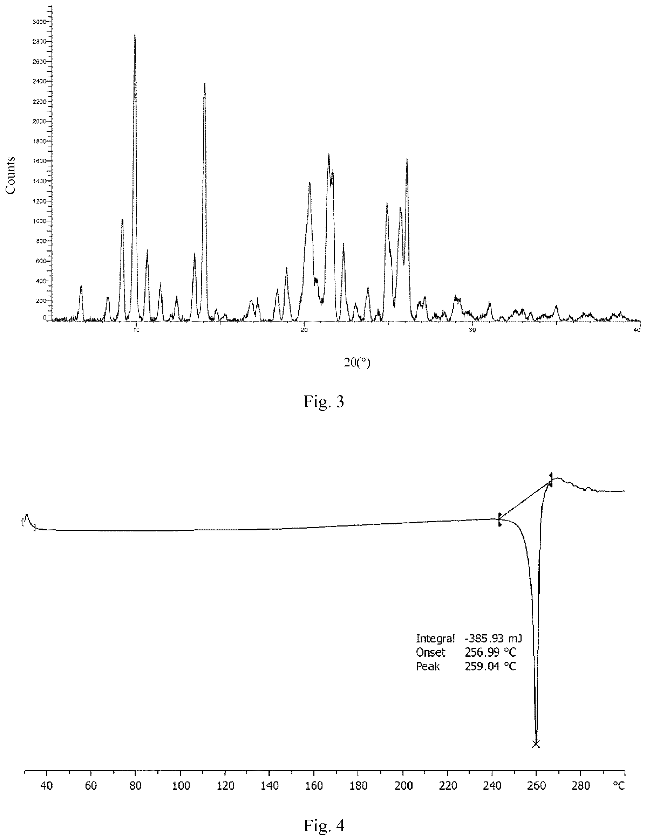

In some embodiments of the present application, an X-ray diffraction pattern of the crystal B of the hydrochloride of the compound represented by formula I according to the application is shown as Fig. 3.

In some embodiments of the present application, a DSC spectrum of the crystal B of the hydrochloride of the compound represented by formula I according to the application has a peak at about 259 °C.

In some embodiments of the present application, a DSC spectrum of the crystal B of the hydrochloride of the compound represented by formula I according to the application is shown as Fig. 4.

In another aspect, the present application provides a method for preparing the crystal B of the hydrochloride of the compound represented by formula I comprising the following steps:
1) contacting the compound represented by formula I with hydrochloric acid; and
2) crystallizing the hydrochloride of the compound represented by formula I from a crystallization solvent, and optionally filtrating the obtained crystal;
wherein the crystallization solvent is ethanol.

In some embodiments of the present application, the molar ratio of hydrochloric acid to the compound represented by formula I in the method for preparing the crystal B of the hydrochloride of the compound represented by formula I is in the range of 1:0.5-1.5, preferably 1:0.8-1.2, and more preferably 1:1.

In some embodiments of the present application, the compound represented by formula I contacts with hydrochloric acid in the crystallization solvent.

In another aspect, the present application provides a crystalline composition of the crystal B of the hydrochloride of the compound represented by formula I. In some embodiments of the present application, the crystal B of the hydrochloride of the compound represented by formula I accounts for 50% or more, preferably 80% or more, more preferably 90% or more, and most preferably 95% or more, by weight of the crystalline composition.

In another aspect, the present application provides a pharmaceutical composition of the crystal B of the hydrochloride of the compound represented by formula I, wherein the pharmaceutical composition comprises a therapeutically effective amount of the crystal B of the hydrochloride of the compound represented by formula I, or the crystalline composition of the crystal B of the hydrochloride of the compound represented by formula I. Furthermore, the pharmaceutical composition may or may not further comprise a pharmaceutically acceptable carrier, excipient, and/or medium.

In another aspect, the present application provides use of the crystal B of the hydrochloride of the compound represented by formula I or the crystalline composition or the pharmaceutical composition as described above in the preparation of a medicament for treating an EGFR-mediated disease.

In another aspect, the present application provides a method for treating an EGFR-mediated disease, comprising administering to a mammal in need thereof a therapeutically effective amount of the crystal B of the hydrochloride of the compound represented by formula I, or the crystalline composition, or the pharmaceutical composition as described above.

In another aspect, the present application provides the crystal B of the hydrochloride of the compound represented by formula I, or the crystalline composition, or the pharmaceutical composition as described above for use in treating an EGFR-mediated disease.

In another aspect, the present application provides a crystal C of the hydrochloride of a compound represented by formula I: 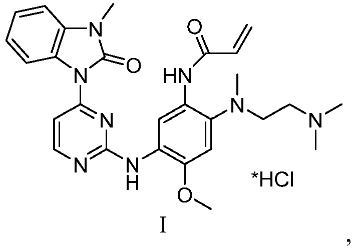 wherein an X-ray diffraction (XRD) pattern of the crystal C of the hydrochloride of the compound represented by formula I has diffraction peaks at 2θ of 7.68°, 8.21°, 10.89°, 15.95°, 19.10°, 20.52° and 21.54°±0.2°; typically has diffraction peaks at 2θ of 7.68°, 8.21°, 9.55°, 10.89°, 15.95°, 19.10°, 20.52°, 21.08°, 21.54° and 28.22°±0.2°; and more typically has diffraction peaks at 2θ of 7.68°, 8.21°, 9.55°, 10.89°, 14.22°, 14.95°, 15.95°, 19.10°, 20.52°, 21.08°, 21.54°, 23.05°, 26.23° and 28.22°±0.2°.

In some embodiments of the present application, X-ray diffraction peaks of the crystal C of the hydrochloride of the compound represented by formula I according to the present application have the following characteristics:

| Serial No. | 2θ±0.2 (°) | Relative Intensity (%) | Serial No. | 2θ±0.2 (°) | Relative Intensity (%) |
|---|---|---|---|---|---|
| 1 | 7.68 | 85.7 | 10 | 20.52 | 43.6 |
| 2 | 8.21 | 80.2 | 11 | 21.08 | 39.2 |
| 3 | 9.55 | 35.6 | 12 | 21.54 | 59.8 |
| 4 | 10.89 | 63.5 | 13 | 22.23 | 15.9 |
| 5 | 14.22 | 24.9 | 14 | 23.05 | 26.1 |
| 6 | 14.95 | 18.2 | 15 | 23.74 | 12.6 |
| 7 | 15.95 | 100.0 | 16 | 26.23 | 29.8 |
| 8 | 19.10 | 53.7 | 17 | 26.99 | 12.5 |
| 9 | 19.77 | 11.0 | 18 | 28.22 | 30.4 |

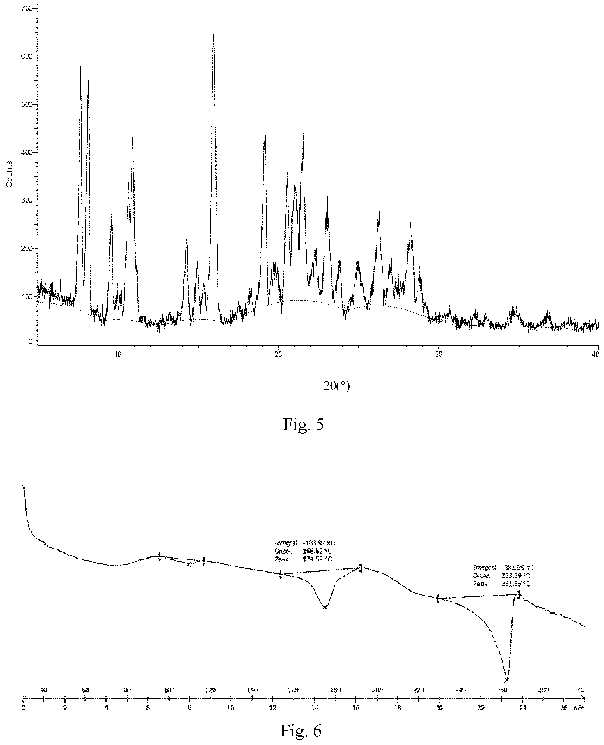

In some embodiments of the present application, an X-ray diffraction pattern of the crystal C of the hydrochloride of the compound represented by formula I according to the application is shown as Fig. 5.

In some embodiments of the present application, a DSC spectrum of the crystal C of the hydrochloride of the compound represented by formula I according to the application has peaks at about 175 °C and 262 °C.

In some embodiments of the present application, a DSC spectrum of the crystal C of the hydrochloride of the compound represented by formula I according to the application is shown as Fig. 6.

In another aspect, the present application provides a method for preparing the crystal C of the hydrochloride of the compound represented by formula I, comprising the following steps:
1) contacting the compound represented by formula I with hydrochloric acid; and
2) crystallizing the hydrochloride of the compound represented by formula I from a crystallization solvent, and optionally filtrating the obtained crystal;
wherein the crystallization solvent is selected from tetrahydrofuran, acetone, or dioxane.

In some embodiments of the present application, the molar ratio of HCl to the compound represented by formula I in the method for preparing the crystal C of the hydrochloride of the compound represented by formula I is in the range of 1:0.5-1.5, preferably 1:0.8-1.2, and more preferably 1:1.

In some embodiments of the present application, the compound represented by formula I contacts with hydrochloric acid in the crystallization solvent.

In another aspect, the present application provides a crystalline composition of the crystal C of the hydrochloride of the compound represented by formula I. In some embodiments of the present application, the crystal C of the hydrochloride of the compound represented by formula I accounts for 50% or more, preferably 80% or more, more preferably 90% or more, and most preferably 95% or more, by weight of the crystalline composition.

In another aspect, the present application provides a pharmaceutical composition of the crystal C of the hydrochloride of the compound represented by formula I, wherein the pharmaceutical composition comprises a therapeutically effective amount of the crystal C of the hydrochloride of the compound represented by formula I, or the crystalline composition of the crystal C of the hydrochloride of the compound represented by formula I. Furthermore, the pharmaceutical composition may or may not further comprise a pharmaceutically acceptable carrier, excipient, and/or medium.

In another aspect, the present application provides use of the crystal C of the hydrochloride of the compound represented by formula I or the crystalline composition or the pharmaceutical composition as described above in the preparation of a medicament for treating an EGFR-mediated disease.

In another aspect, the present application provides a method for treating an EGFR-mediated disease, comprising administering to a mammal in need thereof a therapeutically effective amount of the crystal C of the hydrochloride of the compound represented by formula I, or the crystalline composition, or the pharmaceutical composition as described above.

In another aspect, the present application provides the crystal C of the hydrochloride of the compound represented by formula I, or the crystalline composition, or the pharmaceutical composition as described above for use in treating an EGFR-mediated disease.

In some embodiments of the present application, the EGFR-mediated disease is selected from diseases mediated by EGFR-L858R activating mutations. In some embodiments of the present application, the EGFR-mediated disease is selected from diseases mediated by EGFR-T790M activating mutations. In some embodiments of the present application, the EGFR-mediated disease is selected from diseases mediated by the combined EGFR-L858R and EGFR-T790M activating double mutations. In some embodiments of the present application, the EGFR-mediated disease is a cancer; and the cancer is selected from ovarian cancer, cervical cancer, colorectal cancer, breast cancer, pancreatic cancer, glioma, glioblastoma, melanoma, prostate cancer, leukemia, lymphoma, non-Hodgkin's lymphoma, gastric cancer, lung cancer, hepatocellular carcinoma, gastric cancer, gastrointestinal stromal tumor, thyroid cancer, bile duct cancer, endometrial cancer, kidney cancer, anaplastic large cell lymphoma, acute myeloid leukemia, multiple myeloma, melanoma, or mesothelioma; and the lung cancer may be selected from non-small cell lung cancer, small cell lung cancer, lung adenocarcinoma, or lung squamous cell carcinoma.

The stability of the crystal according to the present application may be detected by placing the crystal under a condition with a high temperature, a high humidity, or a lighting condition. The high temperature condition may be 40 °C to 60 °C, the high humidity condition may be a relative humidity of 75% to 92.5% RH, and the lighting condition may be 5000 Lux. The crystal stability may be evaluated by investigating several parameters, such as the content of the crystal, the total content of impurities, or the water content, of a sample, and comprehensively evaluating these parameters according to the properties of the product.

In the present application, the X-ray diffraction patterns are measured by the following method: instrument: Bruker D2X-ray diffractometer; method: target: Cu; tube voltage: 30 kV; tube current: 10 mA; scan range: 4-40°; scanning speed: 0.1 sec/step, 0.02°/step.

In the present application, the following method for differential scanning calorimetry (DSC) is used: instrument: Mettler DSC-1 differential scanning calorimeter; method: samples (∼5mg) are tested in an aluminum pan for DSC at 30 °C to 300 °C, and at a heating rate of 10 °C/min.

It should be noted that, in an X-ray diffraction spectrum, a diffraction pattern of a crystalline compound is usually characteristic for a specific crystalline form. Relative intensities of the bands (especially at the low angles) can vary depending upon preferential orientation effects resulting from the differences of crystals' conditions, particle sizes, and other measuring conditions. Therefore, the relative intensities of diffraction peaks are not characteristic for a specific crystalline form. It is the relative positions of peaks rather than relative intensities thereof that should be paid more attention when judging whether a crystalline form is the same as a known crystalline form. In addition, as for any given crystalline form, there may be a slight error in the position of peaks, which is also well known in the field of crystallography. For example, the position of a peak may shift due to the change of a temperature, the movement of a sample or the calibration of an instrument and so on when analyzing the sample, and the measurement error of 2θ value is sometimes about ± 0.2°. Accordingly, this error should be taken into consideration when identifying a crystal structure. Usually, the position of a peak is expressed in terms of 2θ angle or lattice spacing d in an XRD pattern and the simple conversion relationship therebetween is d = λ/2sinθ, wherein d represents the lattice spacing, λ represents the wavelength of incident X-ray, and θ represents the diffraction angle. For the same crystalline form of the same compound, the position of peaks in an XRD spectrum thereof has similarity on the whole, and the error of relative intensities may be larger. In addition, it is necessary to point out that due to some factors such as reduced contents, parts of diffraction lines may be absent in the identification of a mixture. At this time, even a band may be characteristic for the given crystalline form without depending upon all the bands of a high purity sample.

It should be noted that DSC is used to measure a thermal transition temperature when absorbing or releasing heat due to the change of a crystal structure or the melting of a crystal. In a continuous analysis of the same crystalline form of the same compound, the error of a thermal transition temperature and a melting point is typically within a range of about ±5°C. When it is said that a compound has a given DSC peak or melting point, it means that the DSC peak or melting point may be varied within a range of ±5°C. DSC provides an auxiliary method to distinguish different crystalline forms. Different crystalline forms can be identified by their characteristically different transition temperatures.

In the present application, the term "pharmaceutical composition" refers to a formulation of one or more compounds of the present application and a carrier, an excipient, and/or a medium generally accepted in the art for transporting a bioactive compound to an organism (e.g., human). An object of the pharmaceutical composition is to facilitate administering the compound of the present application to an organism.

The term "carrier" is defined as a compound that facilitates introducing a compound into a cell or tissue.

The term "pharmaceutically acceptable carrier" includes, but is not limited to, any adjuvant, excipient, glidant, sweetener, diluent, preservative, dye/colorant, flavoring agent, surfactant, wetting agent, dispersant, suspension agent, stabilizer, isotonic agent, solvent, or emulsifier approved by the National Drug Administration as acceptable for use in human or livestocks.

The term "therapeutically effective amount" refers to an amount of the compound of the present application, and when it is administered to a mammal, preferably human, it is enough to realize the treatment of viral infection in a mammal, preferably in human, as defined hereinafter. The amount of the compound of the present application forming the "therapeutically effective amount" changes with the compound, the disease condition and its severity, the administration route, and the age of the mammal to be treated, but can be conventionally determined by those with ordinary skills in the art based on their own knowledge and the disclosure of the present application.

The term "treatment" used herein covers the treatment of viral infection in mammal, preferably viral infection in human, and comprises:
(i) inhibiting viral infection, i.e., arresting its development;
(ii) alleviating viral infection; i.e., causing regression of the viral infection; or
(iii) alleviating symptoms caused by viral infection.

All solvents used in the present application are available on the market, and can be used without further purification. The reactions are generally carried out in an inert nitrogen atmosphere in an anhydrous solvent.

The compounds of the present application are named artificially or named by ChemDraw® software, and vendor directory names are used for the commercially available compounds.

In the present application, the proton nuclear magnetic resonance data are recorded in a BRUKER AVANCE III HD 500M spectrometer; the chemical shift is expressed in ppm downfield from tetramethylsilane; and the mass spectrum is measured by Waters ACQUITY UPLC+XEVO G2 QTof. The mass spectrometer is equipped with an electrospray ion source (ESI) operated in a positive or negative mode.

The crystal A, crystal B and crystal C of the hydrochloride of the compound represented by formula I according to the present application have advantages of high purity, high crystallinity, good stability and so on. Furthermore, the methods for preparing the crystal A, the crystal B and crystal C of the hydrochloride of the compound represented by formula I according to the present application are simple, the solvents used therein are inexpensive and easily available, and the crystallization conditions are mild. Therefore, the methods are suitable for industrial production.

The following examples are provided to further illustrate the technical solutions of the present application in a non-limiting manner. They should not be construed as limiting the scope of the present invention, but merely as illustrative description and typical representatives of the present application. The solvents, reagents, and starting materials used in the present application are chemically pure or analytically pure products available on the market.

### Example 1: N-(2-((2-(dimethylamino)ethyl)(methyl)amino)-4-methoxy-5-(4-(3-methyl-2-oxo-2,3-dihydro-1H-benzo[d]imidazol-1-yl)pyrimidin-2-ylamino)phenyl) acrylamide (I) hydrochloride

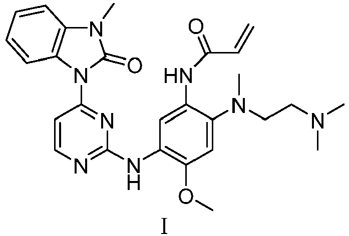

### Step 1: N¹-(2-chloropyrimidin-4-yl)benzene-1,2-diamine

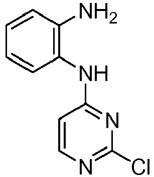

O-phenylenediamine (3.24 g, 30 mmol) and 2,4-dichloropyrimidine (4.47 g, 30 mmol) were dispersed in anhydrous ethanol (60 mL), diisopropylethylamine (7.74 g, 60 mmol) was added, and the resulting mixture was heated to reflux for 3 hours. The solvent was removed by vacuum concentration, and then the residue was dissolved in dichlorometahne (100 mL). The resulting mixture was washed with water, and then washed with a saturated salt solution. The solvent was removed by vacuum concentration. The residue was separated by column chromatography (EA: PE=1: 2) to obtain the title compound (5.32 g, 80 %).
¹H NMR (CDCl₃): δ8.08 (1H, d, J=5.6 Hz), 7.20-7.12 (2H, m), 6.85-6.78 (2H, m), 6.74 (1H, s), 6.24 (1H, d, J=5.6 Hz), 3.82 (2H, br).

### Step 2: 1-(2-chloropyrimidin-4-yl)-1H-benzo[d]imidazol-2(3H)-one

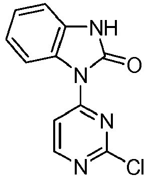

N¹-(2-chloropyrimidin-4-yl)benzene-1,2-diamine (2.21 g, 10 mmol) was dissolved in DMF (15 mL), and carbonyl diimidazole (2.43 g, 15 mmol) was added. The resulting mixture was stirred at room temperature for 1 hour, poured into water (50 mL), and further stirred for another 10 minutes. The resulting mixture was filtered under suction, washed with water (30 mL*3), and dried to obtain the title compound (2.23 g, 90 %).
¹H NMR (DMSO-*d₆*): δ11.64 (1H, br), 8.78 (1H, d, J=5.6 Hz), 8.43 (1H, d, J=5.6 Hz), 8.26 (1H, d, J=7.6 Hz), 7.22-7.10 (3H, m).

### Step 3: 1-(2-chloropyrimidin-4-yl)-3-methyl-1H-benzo[d]imidazol-2(3H)-one

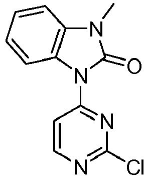

1-(2-Chloropyrimidin-4-yl)-1H-benzo[d]imidazol-2(3H)-one (600 mg, 2.43 mmol) was dispersed in anhydrous DMF (10 mL), and cooled in an ice-water bath. Sodium hydride (116 mg, 60%, 2.90 mmol) was added, and the resulting mixture was stirred for 1 hour. Methyl iodide (345 mg, 2.43 mmol) was added dropwise, and the resulting mixture was further stirred for 1 hour. The reaction mixture was poured into water (50 mL), stirred for 30 minutes, filtered under suction, washed with water (30 mL*3), and dried to obtain the title compound (459 mg, 72%).
¹H NMR (DMSO-*d₆*): δ8.79 (1H, d, J=5.6 Hz), 8.44 (1H, d, J=6.0 Hz), 8.29 (1H, d, J=8.0 Hz), 7.30-7.28 (2H, m), 7.24-7.19 (1H, m), 3.39 (3H, s).

### Step 4: 1 -(2-(4-fluoro-2-methoxy-5 -nitrophenylamino)pyrimidin-4-yl)-3 - methyl-1H-benzo[d]imidazol-2(3H)-one p-toluenesulfonate

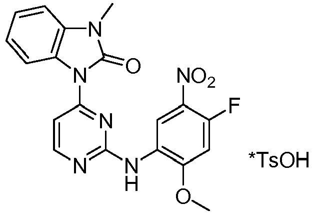

1-(2-Chloropyrimidin-4-yl)-3-methyl-1H-benzo[d]imidazol-2(3H)-one (459 mg, 1.76 mmol), 4-fluoro-2-methoxy-5-nitroaniline (360 mg, 1.93 mmol), and p-toluenesulfonic acid monohydrate (551 mg, 2.89 mmol) were dispersed in 2-pentanol (10 mL). The reaction was stirred at 105 °C overnight. After cooling, the resulting mixture was filtered under suction. The filter cake was washed with a small amount of 2-pentanol three times, and dried to obtain the title compound (440 mg, 43%).
¹H NMR (CDCl₃): δ10.95 (1H, br), 8.49 (1H, d, J=7.6 Hz), 8.39 (1H, d, J=7.2 Hz), 8.21 (1H, d, J=7.2 Hz), 7.87 (2H, d, J=8.4 Hz), 7.68 (1H, d, J=8.4 Hz), 7.28-7.23 (2H, m), 7.04 (2H, d, J=7.6 Hz), 6.91-6.85 (2H, m), 3.92(3H, s), 3.46(3H, s), 2.38(3H, s).

### Step 5: 1-(2-(4-((2-(dimethylamino)ethyl)(methyl)amino)-2-methoxy-5-nitrophenylamino)pyrimidin-4-yl)-3-methyl-1H-benzo[d]imidazol-2(3H)-one

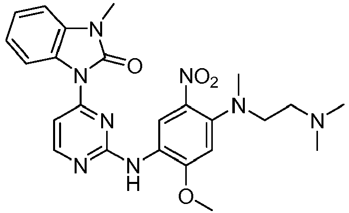

1-(2-(4-Fluoro-2-methoxy-5-nitrophenylamino)pyrimidin-4-yl)-3-methyl-1H-benzo[d]imidazol-2(3H)-one p-toluenesulfonate (440 mg, 0.76 mmol) was dissolved in NMP (5 mL), and diisopropylethylamine (206 mg, 1.59 mmol) and N¹,N¹,N²-trimethylethane-1,2-diamine (116 mg, 1.14 mmol) were added. The reaction was stirred at 85 °C overnight. After cooling, the reaction mixture was poured into water (50 mL), filtered under suction, rinsed with a small amount of methanol, and dried to obtain the title compound (326 mg, 88%).
¹H NMR (CDCl₃): δ8.92 (1H, s), 8.51 (1H, d, J=5.6 Hz), 8.27 (1H, d, J=7.6 Hz), 7.82 (1H, d, J=5.6 Hz), 7.47 (1H, s), 7.29-7.19 (1H, m), 7.17-7.13 (1H, m), 7.04 (1H, d, J=7.6 Hz), 6.69 (1H, s), 3.98(3H, s), 3.47(3H, s), 3.27 (2H, t, J=7.2Hz), 2.89 (3H, s), 2.88 (2H, t, J=7.2Hz), 2.26 (6H, s).

### Step 6: 1-(2-(5-amino-4-((2-(dimethylamino)ethyl)(methyl)amino)-2-methoxyphenylamino)pyrimidin-4-yl)-3-methyl-1H-benzo[d]imidazol-2(3H)-one

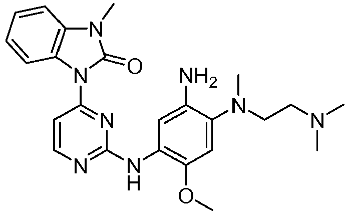

1-(2-(4-((2-(Dimethylamino)ethyl)(methyl)amino)-2-methoxy-5-nitrophenyl amino)pyrimidin-4-yl)-3-methyl-1H-benzo[d]imidazol-2(3H)-one (326 mg, 0.66 mmol) was dissolved in methanol (10 mL), Pd/C (10%, 30 mg) was added, and air was replaced with hydrogen gas three times. The system was stirred in a hydrogen gas atmosphere overnight, and filtered under suction. The product was easy to be oxidized. The resulting filtrate was rapidly concentrated under vacuum, and directly used in the next reaction.

### Step 7: N-(2-((2-(dimethylamino)ethyl)(methyl)amino)-4-methoxy-5-(4-(3-methyl-2-oxo-2,3-dihydro-1H-benzo[d]imidazol-1-yl)pyrimidin-2-ylamino)phenyl) acrylamide hydrochloride

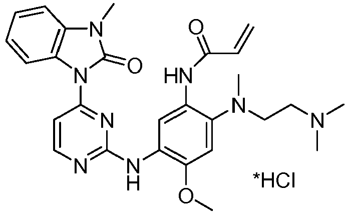

1-(2-(5-Amino-4-((2-(dimethylamino)ethyl)(methyl)amino)-2-methoxyphenyl amino)pyrimidin-4-yl)-3-methyl-1H-benzo[d]imidazol-2(3H)-one obtained in the previous reaction was dissolved in anhydrous dichloromethane (10 mL), diisopropylethylamine (129 mg, 1.00 mmol) was added, and the resulting mixture was cooled in an ice-water bath. A solution of acryloyl chloride (60 mg, 0.66 mmol) in anhydrous dichloromethane (2 mL) was slowly added to the system dropwise in 15 minutes. After further stirring for 15 minutes, the reaction mixture was poured into petroleum ether (50 mL), and stirred for 10 minutes. After suction filtration, the filter cake was rinsed with petroleum ether. The resulting crude product was separated by column chromatography (DCM: MeOH=20: 1) to obtain the title compound (164 mg, total yield in the two steps: 45 %).
¹H NMR (DMSO-*d₆*): δ10.15 (1H, br), 9.72 (1H, br), 8.70 (1H, s), 8.41 (1H, d, J=5.6 Hz), 8.16-8.12 (2H, m), 7.67 (1H, d, J=5.6 Hz), 7.22-7.12 (2H, m), 6.99-6.92 (3H, m), 6.19 (1H, dd, J=2.0 Hz, 17.2 Hz), 5.68 (1H, dd, J=2.0Hz, 10.4 Hz), 3.77 (3H, s), 3.34 (3H, s), 3. 28 (4H, br), 2.72 (6H, s), 2.60 (3H, s).

### Example 2: N-(2-((2-(dimethylamino)ethyl)(methyl)amino)-4-methoxy-5-(4-(3-methyl-2-oxo-2,3-dihydro-1H-benzo[d]imidazol-1-yl)pyrimidin-2-ylamino)phenyl) acrylamide (I)

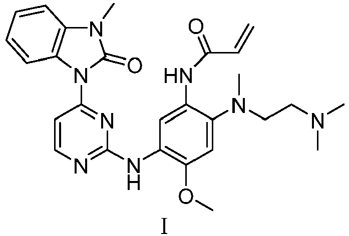

1-(2-(5-Amino-4-((2-(dimethylamino)ethyl)(methyl)amino)-2-methoxyphenyl amino)pyrimidin-4-yl)-3-methyl-1H-benzo[d]imidazol-2(3H)-one (82 g) obtained in Step 6 of Example 1 was dissolved in THF (800 mL) and water (80 mL) under stirring, and 3-chloropropionyl chloride (24.8 g) was added dropwise. After TLC showed that the starting material disappeared, triethylamine (358.2 g) was added, and the resulting mixture was heated to 65 °C. After the reaction was completed, the reaction mixture was concentrated to dryness. The residue was dissolved in 1L of dichloromethane, and stratified with water (500mL) twice. The organic phases were collected and concentrated to obtain 88 g of a crude product. The resulting crude product was separated by column chromatography (DCM: MeOH=20: 1) to obtain the title compound (62.5g).
ESI-MS [M+H]⁺: 517.2677.
¹H NMR (DMSO-*d₆*): δ10.05 (1H, s), 8.67 (1H, s), 8.5 (1H, s), 8.44 (1H, d, J=5.6 Hz), 8.12 (1H, d, J=7.6 Hz), 7.13 (2H, m), 6.9 (1H, t, J=6.4 Hz), 7.7 (1H, d, J=5.6 Hz), 7.05 (1H, s), 6.4 (1H, dd, J=10.15Hz, 16.9 Hz), 6.21 (1H, dd, J=1.6Hz, 16.9 Hz), 5.72 (1H, brd, J=11.50 Hz), 3.77 (3H, s), 3.35 (3H, s), 2.91 (2H, t, J=5.65 Hz), 2.75 (3H, s), 2.34 (2H, t, J=5.7 Hz), 2.21 (6H, s).

### Example 3: Crystal A of the hydrochloride of a compound represented by formula I

### Method 1

10 g of the compound obtained in Example 2 was added to a 500 mL reactor. 150 mL of ethanol was added, and stirred sufficiently to obtain a homogeneous system. 10 mL of 2N hydrochloric acid was slowly added. After a clear solution was obtained, the resulting solution was stirred for 2 hours, and filtered. The filter cake was dried under vacuum at 45-50 °C. The collected solid (8.3 g) was dissolved in 49.8 mL of a solution of ethanol and water (ethanol:water=3:1). The system was stirred at 80 °C to obtain a clear solution, and then the solution was cooled to 25-30 °C, and filtered. The filter cake was dried under vacuum at 45-50 °C, to obtain a corresponding crystal.

### Method 2

1g of the compound obtained in Example 2 was added to a 25 mL reactor. 10 mL of acetonitrile was added, and the resulting mixture was stirred sufficiently to obtain a homogeneous system. 1 mL of 2N hydrochloric acid was slowly added, and the solid was dissolved gradually to obtain a clear solution. After further stirring for 10 min, solids precipitated. After fully stirring for 12 hours, the resulting mixture was filtered. The filter cake was rinsed with 2 mL of acetonitrile, and dried under vacuum at 45 °C, to obtain a corresponding crystal.

### Method 3

1 g of the compound obtained in Example 2 was added to a 25 mL reactor. 5 mL of methanol was added, and the resulting mixture was stirred sufficiently to obtain a homogeneous system. 1 mL of 2N hydrochloric acid was slowly added, and the solid was dissolved gradually to obtain a clear solution. After further stirring for 10 min, solids precipitated. After fully stirring for 12 hours, the resulting mixture was filtered. The filter cake was rinsed with 2 mL of methanol, and dried under vacuum at 45 °C, to obtain a corresponding crystal.

### Method 4

1 g of the compound obtained in Example 2 was added to a 25 mL reactor. 5 mL of isopropanol was added, and the resulting mixture was stirred sufficiently to obtain a homogeneous system. 1 mL of 2N hydrochloric acid was slowly added, and the solid was dissolved gradually to obtain a clear solution. Solids precipitated very soon. After fully stirring for 12 hours, the resulting mixture was filtered. The filter cake was rinsed with 2 mL of isopropanol, and dried under vacuum at 45 °C, to obtain a corresponding crystal.

### Example 4: Crystal B of the hydrochloride of a compound represented by formula I

### Method 5

10 g of the compound obtained in Example 2 was added to a 500 mL reactor. 150 mL of ethanol was added, and the resulting mixture was stirred at 25 °C. At this moment, the reaction system did not form a clear solution. 2N hydrochloric acid was slowly added, and the resulting mixture was stirred for 2 hours, and filtered. The filter cake was dried under vacuum at 45-50 °C to obtain the desired crystal form.

### Example 5: Crystal C of the hydrochloride of a compound represented by formula I

### Method 6

1 g of the compound obtained in Example 2 was added to a 25 mL reactor. 5 mL of tetrahydrofuran was added, and the resulting mixture was stirred sufficiently to obtain a homogeneous system. 1 mL of 2N hydrochloric acid was slowly added, the solid was dissolved gradually to obtain a clear solution, and the solution became cloudy soon. After fully stirring for 12 hours, the resulting mixture was filtered. The filter cake was rinsed with 2 mL of tetrahydrofuran, and dried under vacuum at 45 °C, to obtain a corresponding crystal.

### Method 7

1 g of the compound obtained in Example 2 was added to a 25 mL reactor. 5 mL of acetone was added, and the resulting mixture was stirred sufficiently to obtain a homogeneous system. 1 mL of 2N hydrochloric acid was slowly added, and the solid was dissolved gradually to obtain a clear solution. After further stirring for 10 min, solids precipitated. After fully stirring for 12 hours, the resulting mixture was filtered. The filter cake was rinsed with 2 mL of acetone, and dried under vacuum at 45 °C, to obtain a corresponding crystal.

### Method 8

1 g of the compound obtained in Example 2 was added to a 25 mL reactor. 5 mL of 1,4-dioxane was added, and the resulting mixture was stirred sufficiently to obtain a homogeneous system. 1 mL of 2N hydrochloric acid was slowly added, and the solid was dissolved gradually to obtain a clear solution. After further stirring for 10 min, solids precipitated. After fully stirring for 12 hours, the resulting mixture was filtered. The filter cake was rinsed with 2 mL of 1,4-dioxane, and dried under vacuum at 45 °C, to obtain a corresponding crystal.

### Example 6: Stability Test

The crystal A obtained by Method 1 of Example 3 was kept away from light at room temperature, and sampled for detection in months 1.5, 2, 5, and 6, respectively. The detection results were compared with the initial detection result on day 0, and the test results were shown in the table below:

| Items | Room Temperature, Kept Away From Light | | | | |
|---|---|---|---|---|---|
| | Initial Result | Month 1.5 | Month 2 | Month 5 | Month 6 |
| Characters | off-white powder | off-white powder | off-white powder | off-white powder | off-white powder |
| Content (%) | 100.3% | 100.5% | / | 100% | 99.8% |
| Total Impurity (%) | 1.11% | 1.52% | 1.23% | 1.5% | 1.37% |

The crystal B obtained by Method 5 of Example 4 was kept respectively in an environment at a high temperature of 60 °C, a high humidity of 75% RH, a high humidity of 92.5% RH, or an illumination intensity of 5000 Lux, and sampled for detection on days 5, 10, and 30, respectively. The detection results were compared with the initial detection result on day 0, and the test results were shown in the table below:

| Research Items | Day 0 | High Temp. 60 °C | | | High Humidity 75% | | | High Humidity 92.5% | | | Illumination 5000 Lux | | |
|---|---|---|---|---|---|---|---|---|---|---|---|---|---|
| | | Day 5 | Day 10 | Day 30 | Day 5 | Day 10 | Day 30 | Day 5 | Day 10 | Day 30 | Day 5 | Day 10 | Day 30 |
| Characters | white powder | white powder | white powder | off-white powder | white powder | white powder | off-white powder | white powder | white powder | off-white powder | white powder | white powder | off-white powder |
| Content (%) | 99.3 | 100.5 | 104.2 | 107.9 | 99.3 | 103.6 | 106.4 | 98.4 | 105.2 | 103.9 | 99.4 | 106.9 | 107.9 |
| Total Impurity (%) | 0.12 | / | 0.09 | 0.19 | / | 0.07 | 0.06 | / | 0.06 | 0.07 | 0.06 | 0.14 | 0.37 |

### Example 7: In vitro Activity Assays

### 1. Method of in vitro enzymatic assay

EGFR or EGFR (T790M, L858R) kinase was obtained by being expressed and purified through an insect expression system, or purchased as commercially available products.

A platform for testing the activities of EGFR or EGFR (T790M, L858R) kinase was established based on the Homogeneous Time-Resolved Fluorescence (HTRF) method provided by Cisbio Inc., and was used for determining the activities of compounds. The compounds were diluted at a 10-fold gradient with 100% DMSO with a starting concentration of 1 µM. 4 µl of each concentration was taken and added to 96 µl of reaction buffer (50 mM HEPES (pH 7.0), 0.02% NaN₃, 0.01% BSA, 0.1 mM Orthovanadate, 5 mM MgCl₂, 50 nM SEB, 1 mM DTT). 2.5 µl of the mixture was taken and added to a 384-well plate (OptiPlate-384, PerkinElmer), and then 2.5 µl of the kinase was added. After thoroughly mixing by centrifugation, 5 µl of ATP and TK Substrate-biotin was added to initiate the reaction. The 384-well plate was incubated in an incubator at 23°C for a period of time, and then the reaction was terminated by adding 5 µl of Eu3+-Cryptate labeled TK-Antibody and 5 µl of streptavidin-XL665. The fluorescence values were read on Envision (PerkinElmer) after incubating in the incubator for 1 hour. The IC₅₀ values of the compounds were calculated using the GraphPad Prism 5.0 software.

### 2. Cell proliferation assay

Human non-small cell lung cancer cells NCI-H1975 were cultured in RPIM-1640 culture medium supplemented with 10% fetal bovine serum and 1% penicillin-plus-streptomycin in a cell incubator (37°C, 5% CO₂). The cells were seeded in a 96-well plate at a density of 2,000 cells per well (volume: 195 µl) and cultured overnight. On the next day, the compounds were added. In particular, the compounds were diluted at a 3-fold gradient with a starting concentration of 10 mM. 4 µl of each concentration was taken and added into 96 µl of culture medium. Then, 5 µl of the mixture was taken and added to a cell culture medium (final DMSO concentration being 0.1%, v/v). After treatment for 72 hours, the medium was aspirated and 30 µl of CellTiter-Glo® (Promega) reagent was added. Fluorescence signals were read on Envison (Perkin Elmer), and IC₅₀ values of the compounds for inhibiting cell proliferation were calculated using GraphPad Prism 5.0.

Human skin squamous carcinoma cell line A431 was cultured in DMEM supplemented with 10% fetal bovine serum and 1% penicillin-plus-streptomycin in a cell incubator (37°C, 5% CO₂). In the tests of the compounds, the bottom substrate was at a concentration of 0.6%. Cells were re-suspended with 0.3% low-melting-point agar, and then seeded in a 96-well plate at a density of 2,000 cells per well (100 µl). The compounds were diluted at a 3-fold gradient with a starting concentration of 10 mM. 2 µl of each concentration was taken and added to 98 µl of culture medium, and then 5.3 µl of the mixture was added to the cell culture medium (final DMSO concentration being 0.1%, v/v). After treatment for one week (7 days), 20 µl of CellTiter-Blue® (Promega) reagent was added, and the plate was incubated at 37°C for 4 hours. Fluorescence signals were read on Envison (Perkin Elmer), and IC₅₀ values of the compound for inhibiting cell proliferation were calculated using GraphPad Prism 5.0.

**Biological Activity List**

| Compound | Enzyme Activity (IC₅₀ nM) | | | Cell Viability (IC₅₀ nM) | |
|---|---|---|---|---|---|
| | EGFR (WT) | EGFR-L858R/T790M (DM) | WT/DM | A431 | NCI-H1975 |
| AZD9291 | 19.45 | 2.04 | 9.5 | 53.54 | 9.08 |
| Example 1 | 9.07 | 0.72 | 12.6 | 22.49 | 2.76 |

AZD9291 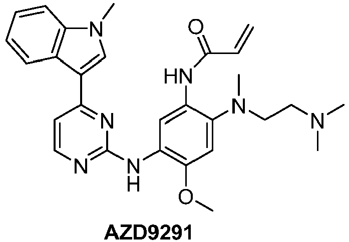 was obtained according to Example 28 in WO2013014448.

### Example 8: Evaluation on Pharmacokinetics

The test compound was intragastrically administered to healthy adult male rats at a single dose of 10 mg/kg (adjuvant: 20% sulfobutyl ether-β-cyclodextrin). The animals were fasted overnight prior to the experiment, i.e., fasted from 10 hours prior to intragastric administration to 4 h after administration, and blood samples were collected in hours 0.25, 0.5, 1, 2, 4, 6, 8, and 24 after intragastric administration. About 0.3mL of whole blood was collected from the orbital venous plexus, and put in a heparin anticoagulant tube. The sample was centrifuged at 4 °C at 4000 rpm for 5 min. The plasma was transferred to a centrifuge tube, and kept at -80 °C until analysis. Concentration of the test product in the plasma sample was analyzed using non-validated liquid chromatography-tandem mass spectrometry (LC-MS/MS). Plasma concentration-time data of individual animals were analyzed using WinNonlin (Professional Edition 6.3; Pharsight Corporation) software. A non-compartment model was used for concentration analysis. Pharmacokinetic parameters of the test compound were calculated.

| Parameter | Unit | PO 10mg/kg | |
|---|---|---|---|
| | | Example 2 | Example 1 |
| t_{1/2} | hr | 2.45 | 1.12 |
| Tₘₐₓ | hr | 0.67 | 0.67 |
| Cₘₐₓ | ng/mL | 94.4 | 272 |
| AUC_{0-INF} | hr*ng/mL | 401 | 667 |

## Claims

1. A crystal A of the hydrochloride of a compound represented by formula I: 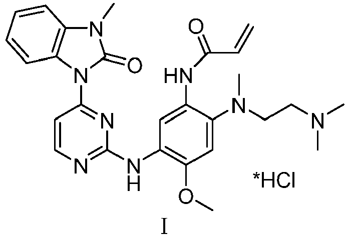 wherein an X-ray diffraction pattern of the crystal A of the hydrochloride of the compound represented by formula I has diffraction peaks at 2θ of 8.96°±0.2°, 14.11°±0.2°, 14.87°±0.2°, 16.52°±0.2°, 18.67°±0.2°, 21.93°±0.2° and 27.09°±0.2°; typically has diffraction peaks at 2θ of 8.33°±0.2°, 8.96°±0.2°, 12.16°±0.2°, 14.11°±0.2°, 14.87°±0.2°, 16.52°±0.2°, 17.66°±0.2°, 18.67°±0.2°, 21.93°±0.2° and 27.09°±0.2°; more typically has diffraction peaks at 2θ of 8.33°±0.2°, 8.96°±0.2°, 11.74°±0.2°, 12.16°±0.2°, 14.11°±0.2°, 14.87°±0.2°, 16.52°±0.2°, 17.66°±0.2°, 18.23°±0.2°, 18.67°±0.2°, 21.93°±0.2°, 22.65°±0.2° and 27.09°±0.2°; and further typically has diffraction peaks at 2θ of 8.33°±0.2°, 8.96°±0.2°, 11.74°±0.2°, 12.16°±0.2°, 14.11°±0.2°, 14.87°±0.2°, 16.52°±0.2°, 17.66°±0.2°, 18.23°±0.2°, 18.67°±0.2°, 19.48°±0.2°, 19.92°±0.2°, 21.93°±0.2°, 22.65°±0.2°, 24.95°±0.2°, 27.09°±0.2° and 27.55°±0.2°.

2. The crystal A of the hydrochloride of the compound represented by formula I according to claim 1, wherein its DSC spectrum has a peak at 271 °C.

3. A method for preparing the crystal A of the hydrochloride of the compound represented by formula I according to claim 1, comprising the following steps:
1) contacting the compound represented by formula I with hydrochloric acid; and
2) crystallizing the hydrochloride of the compound represented by formula I from a crystallization solvent, and optionally filtrating the obtained crystal;
wherein the crystallization solvent is selected from acetonitrile, methanol, isopropanol, or a mixture of ethanol and water.

4. A crystalline composition, wherein the crystal A of the hydrochloride of the compound represented by formula I according to claim 1 or 2 accounts for 50% or more, preferably 80% or more, more preferably 90% or more, and most preferably 95% or more, by weight of the crystalline composition.

5. A pharmaceutical composition, comprising a therapeutically effective amount of the crystal A of the hydrochloride of the compound represented by formula I according to claim 1 or 2, or the crystalline composition according to claim 4.

6. Use of the crystal A of the hydrochloride of the compound represented by formula I according to claim 1 or 2, or the crystalline composition according to claim 4, or the pharmaceutical composition according to claim 5 in the preparation of a medicament for treating an EGFR-mediated disease.

7. A crystal B of the hydrochloride of a compound represented by formula I: 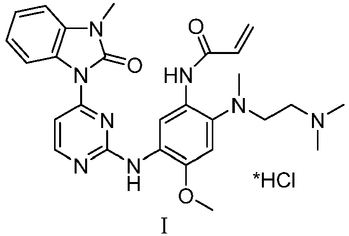 wherein an X-ray diffraction pattern of the crystal B of the hydrochloride of the compound represented by formula I has diffraction peaks at 2θ of 9.17°±0.2°, 9.93°±0.2°, 14.07°±0.2°, 20.31°±0.2°, 21.44°±0.2° and 26.10°±0.2°; typically has diffraction peaks at 2θ of 9.17°±0.2°, 9.93°±0.2°, 10.65°±0.2°, 13.46°±0.2°, 14.07°±0.2°, 20.31°±0.2°, 21.44°±0.2°, 22.33°±0.2°, 24.93°±0.2° and 26.10°±0.2°; and more typically has diffraction peaks at 2θ of 6.71°±0.2°, 9.17°±0.2°, 9.93°±0.2°, 10.65°±0.2°, 11.44°±0.2°, 13.46°±0.2°, 14.07°±0.2°, 18.94°±0.2°, 20.31°±0.2°, 21.44°±0.2°, 21.66°±0.2°, 22.33°±0.2°, 24.93°±0.2°, 25.73°±0.2° and 26.10°±0.2°.

8. The crystal B of the hydrochloride of the compound represented by formula I according to claim 7, wherein its DSC spectrum has a peak at 259 °C.

9. A method for preparing the crystal B of the hydrochloride of the compound represented by formula I according to claim 7, comprising the following steps:
1) contacting the compound represented by formula I with hydrochloric acid; and
2) crystallizing the hydrochloride of the compound represented by formula I from a crystallization solvent, and optionally filtrating the obtained crystal;
wherein the crystallization solvent is ethanol.

10. A crystalline composition, wherein the crystal B of the hydrochloride of the compound represented by formula I according to claim 7 or 8 accounts for 50% or more, preferably 80% or more, more preferably 90% or more, and most preferably 95% or more, by weight of the crystalline composition.

11. A pharmaceutical composition, comprising a therapeutically effective amount of the crystal B of the hydrochloride of the compound represented by formula I according to claim 7 or 8, or the crystalline composition according to claim 10.

12. Use of the crystal B of the hydrochloride of the compound represented by formula I according to claim 7 or 8, or the crystalline composition according to claim 10, or the pharmaceutical composition according to claim 11 in the preparation of a medicament for treating an EGFR-mediated disease.

13. A crystal C of the hydrochloride of a compound represented by formula I: 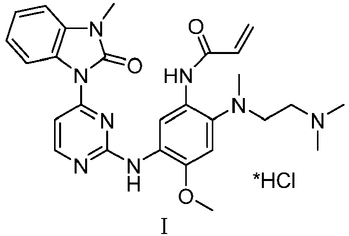 wherein an X-ray diffraction pattern of the crystal C of the hydrochloride of the compound represented by formula I has diffraction peaks at 2θ of 7.68°±0.2°, 8.21°±0.2°, 10.89°±0.2°, 15.95°±0.2°, 19.10°±0.2°, 20.52°±0.2° and 21.54°±0.2°; typically has diffraction peaks at 2θ of 7.68°±0.2°, 8.21°±0.2°, 9.55°±0.2°, 10.89°±0.2°, 15.95°±0.2°, 19.10°±0.2°, 20.52°±0.2°, 21.08°±0.2°, 21.54°±0.2° and 28.22°±0.2°; and more typically has diffraction peaks at 2θ of 7.68°±0.2°, 8.21°±0.2°, 9.55°±0.2°, 10.89°±0.2°, 14.22°±0.2°, 14.95°±0.2°, 15.95°±0.2°, 19.10°±0.2°, 20.52°±0.2°, 21.08°±0.2°, 21.54°±0.2°, 23.05°±0.2°, 26.23°±0.2° and 28.22°±0.2°.

14. The crystal C of the hydrochloride of the compound represented by formula I according to claim 13, wherein its DSC spectrum has peaks at 175 °C and 262 °C.

15. A method for preparing the crystal C of the hydrochloride of the compound represented by formula I according to claim 13, comprising the following steps:
1) contacting the compound represented by formula I with hydrochloric acid; and
2) crystallizing the hydrochloride of the compound represented by formula I from a crystallization solvent, and optionally filtrating the obtained crystal;
wherein the crystallization solvent is selected from tetrahydrofuran, acetone, or dioxane.

16. A crystalline composition, wherein the crystal C of the hydrochloride of the compound represented by formula I according to claim 13 or 14 accounts for 50% or more, preferably 80% or more, more preferably 90% or more, and most preferably 95% or more, by weight of the crystalline composition.

17. A pharmaceutical composition, comprising a therapeutically effective amount of the crystal C of the hydrochloride of the compound represented by formula I according to claim 13 or 14, or the crystalline composition according to claim 16.

18. Use of the crystal C of the hydrochloride of the compound represented by formula I according to claim 13 or 14, or the crystalline composition according to claim 16, or the pharmaceutical composition according to claim 17 in the preparation of a medicament for treating an EGFR-mediated disease.
